# FASCICULE DE BREVET EUROPEEN

(11) **EP 0 363 389 B1**
(45) Date de publication et mention de la délivrance du brevet: **26.01.1994**
(21) Numéro de dépôt: 88904393.1
(22) Date de dépôt: 28.04.1988
(51) Int. Cl.: A61B 17/16, A61C 8/00

(54) **FIXATEUR EXTERNE POUR CHIRURGIE OSSEUSE**
EXTERNE FIXIERVORRICHTUNG FÜR KNOCHENCHIRURGIE
EXTERNAL FIXING DEVICE FOR BONE SURGERY

(30) Priorité: 14.05.1987 BE 8700535
(43) Date de publication de la demande: 18.04.1990
(73) Titulaire: Dury, Georges Emile Ladislas, B-1050 Bruxelles (BE)
(72) Inventeur: Dury, Georges Emile Ladislas, B-1050 Bruxelles (BE)
(74) Mandataire: Vigneron, Jean
(86) Numéro de dépôt international: BE8800012
(87) Numéro de publication internationale: WO8808691

(56) Documents cités:
- EP-A- 0 215 765
- FR-A- 2 412 302
- US-A- 3 760 504
- US-A- 4 257 411

## Description

La présente invention a pour objet un fixateur externe pour chirurgie osseuse, en particulier dento-maxilo-faciale, et notamment pour la réalisation de trépanations permettant la mise en place d'implants.

Du document EP-A-0 215 765 est déjà connu un fixateur externe pour chirurgie osseuse, en particulier dento-maxilo-faciale, et notamment pour la réalisation de trépanations permettant la mise enplace d'implants, comprenant au moins une tige indéformable servant notamment de poignée au fixateur et présentant une partie de section constante et d'axe rectiligne ; un élément écarteur, tel qu'écarteur de lèvre, destiné à prendre appui sur une première face de l'os à trépaner, qui est fixé à la tige et sensiblement perpendiculairement à l'axe de la partie susdite de cette derniére ; un élément associé à la tige précitée et opposée à l'élément écarteur, une ouverture étant pratiquée dans cet élément opposé, l'axe de laquelle est sensiblement parallèle à l'axe de la partie de section constante de tige et laquelle est destinée à livrer passage à un outil de trépanation de l'os, tel qu'une fraise ; voir en particulier la figure 13 du document précité.

L'invention a pour but de procurer un fixateur externe, du genre susdit, pouvant être équipé de différents accessoires qui permettent de réaliser avec une très grande précision toutes les trépanations nécessaires pour la pose d'implants et ce, quel que soit le type de ces derniers.

A cet effet, suivant l'invention, ce fixateur externe comprend au moins une tige indéformable servant notamment de poignée au fixateur et présentant une partie de section constante et d'axe rectiligne, un élément écarteur, tel qu'écarteur de lèvre, destiné à prendre appui sur une première face de l'os à trépaner, qui est fixé à la tige à distance des extrémités de la partie susdite de cette dernière et sensiblement perpendiculairement à l'axe de cette partie de tige, au moins un élément mobile, associé à la partie de tige précitée de manière à pouvoir coulisser librement le long de cette dernière pour prendre appui soit sur la muqueuse couvrant une seconde face de l'os opposée à la première avec laquelle coopère l'élément écarteur ou sur une plaque de protection de cette muqueuse, soit avec cette seconde face de l'os, dans lequel est pratiquée une ouverture, d'axe sensiblement parallèle à l'axe de la partie de tige, destinée à livrer passage à un outil de trépanation de l'os, tel qu'une fraise, et des moyens prévus sur ledit élément pour l'immobiliser en position choisie par rapport à la partie de tige précitée.

Suivant un mode de réalisation de l'invention, ledit fixateur comprend un support mobile pour un outil de trépanation, tel que contre-angle porte-fraise, agencé pour coulisser librement le long de la partie de tige précitée à l'opposé de l'élément écarteur par rapport à l'élément mobile susdit, des moyens agencés pour immobiliser le support mobile par rapport à la tige en position choisie, des moyens pour emprisonner le contre-angle porte-fraise et des moyens pour l'immobiliser par rapport audit support mobile pour que la fraise et l'ouverture réalisée dans l'élément écarteur soient coaxiales.

Suivant une forme de réalisation avantageuse de l'invention, le fixateur externe comprend un organe mobile destiné à supporter un guide pour l'outil de trépanation, afin que ce guide et l'ouverture pratiquée dans l'élément écarteur soient coaxiaux, cet organe coulissant librement sur la partie précitée de tige à l'opposé de l'élément mobile par rapport à l'élément écarteur et présentant des moyens agencés pour l'immobiliser sur la partie de tige en position choisie.

Suivant un mode de réalisation particulièrement avantageux de l'invention, ledit fixateur externe comprend, d'une part, un support mobile pour outil de trépanation, tel que pièce à main porte-fraise, agencé pour coulisser librement le long de la partie de tige précitée à l'opposé de l'élément écarteur par rapport à l'organe mobile susdit, des moyens agencés pour immobiliser ce support, en position choisie, par rapport à la partie de tige précitée et des moyens agencés pour immobiliser l'outil de trépanation pour que son axe coïncide avec l'axe de l'ouverture pratiquée dans l'élément écarteur et, d'autre part, un support mobile pour au moins un outil de trépanation, tel que fraise, agencé pour coulisser librement le long de la partie de tige précitée dans sa section comprise entre l'élément écarteur et l'élément mobile précités, des moyens agencés pour immobiliser ce support, en position choisie, par rapport à la partie de tige et des moyens pour immobiliser l'outil de trépanation dans le support pour que son axe soit transversal à l'axe de l'ouverture pratiquée dans l'élément écarteur.

D'autres détails et particularités de l'invention ressortiront de la description des dessins annexés au présent mémoire et qui représentent, à titre d'exemples non limitatifs, des formes de réalisation particulières du fixateur externe suivant l'invention.

La figure 1 est une vue en perspective, avec brisures partielles, montrant le fixateur externe suivant l'invention dans sa forme de réalisation la plus simple.

La figure 2 est une vue en perspective, avec brisures partielles montrant une variante du fixateur externe illustré à la figure 1.

Les figures 3 à 5 sont des vues analogues à la figure 2 montrant, avec brisures partielles, d'une part, le fixateur externe équipé de différents accessoires et, d'autre part, des détails des éléments constitutifs dudit fixateur.

La figure 6 est une vue en perspective illustrant une variante du fixateur externe représenté aux figures 3 à 5.

La figure 7 est une vue partielle, en élévation, montrant un autre agencement du fixateur représenté à la figure 6.

Dans les différentes figures, les mêmes notations de référence désignent des éléments identiques ou analogues.

Le fixateur externe 1 suivant l'invention et illustré aux dessins est tout particulièrement destiné à permettre la réalisation aisée et extrêmement précise de trépanations, en chirurgie dento-maxilo-faciale en vue de la mise en place d'implants.

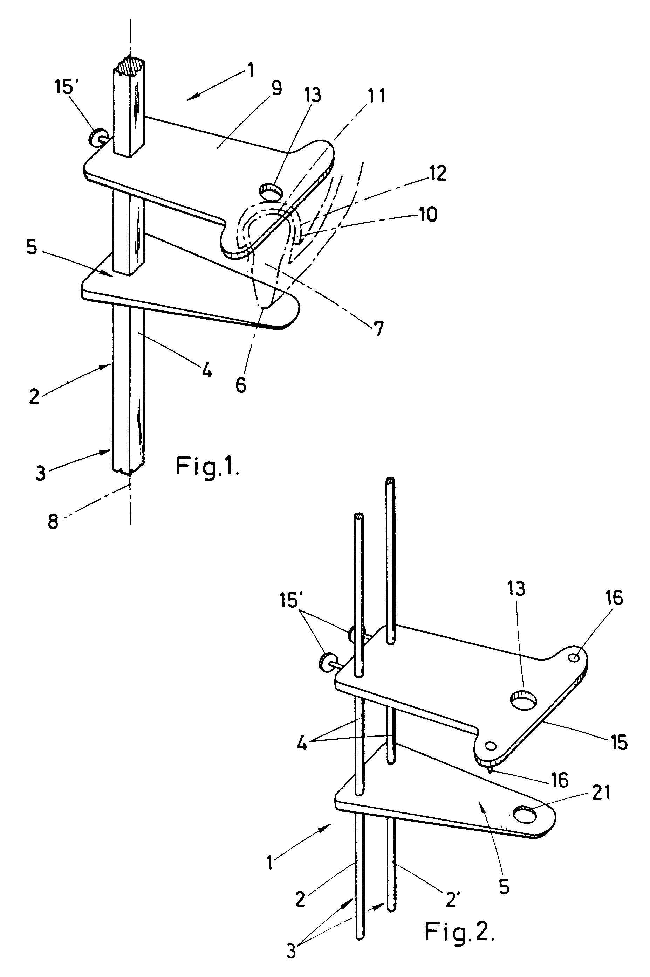

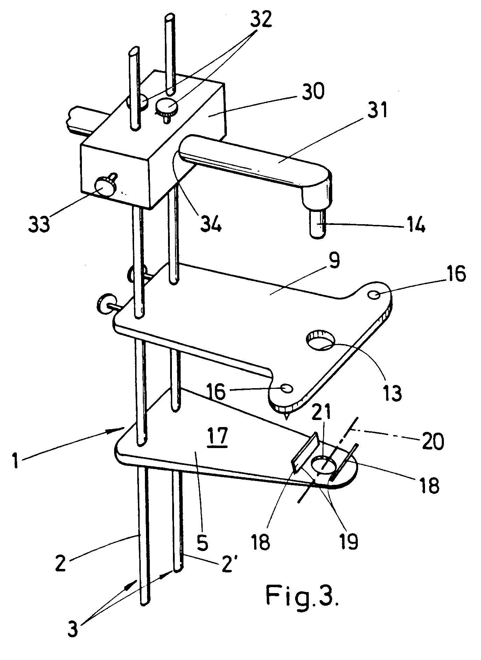

Dans sa forme de réalisation la plus simplifiée, illustrée aux figures 1 et 2, le fixateur externe 1 comprend, par exemple pour la pose d'implants simples du type tige, soit une tige 2 (figure 1), soit deux tiges parallèles 2 et 2' (figure 2) indéformables, dont l'extrémité 3 peut notamment servir de poignée au fixateur et qui présentent chacune une partie 4 de section constante et rectiligne. Sur cette partie 4 de la ou des tiges 2 et 2' est fixé, à distance des extrémités de ladite partie 4, un élément 5 écarteur de lèvre destiné à prendre appui sur une première face 6 de l'os à trépaner 7, représenté schématiquement en traits interrompus à la figure 1, et qui s'étend sensiblement perpendiculairement à l'axe 8 de cette partie 4 de tige. Cette dernière supporte également un élément mobile 9 qui coulisse librement suivant l'axe 8 pour prendre appui soit sur la muqueuse 10 couvrant la face 11 de l'os opposée à la face 6 susdite ou sur une plaque de protection 12, représentée en traits interrompus à la figure 1, de ladite muqueuse, soit sur la face 11 de l'os. Cet élément 9 présente une ouverture 13, d'axe parallèle à l'axe 8 susdit, destinée à livrer passage à un outil de trépanation 14 (figure 3), tel que fraise, ainsi qu'une ou des vis de pression 15' prévues pour l'immobiliser en position choisie, par rapport à la partie 4 de la tige 2 ou des tiges parallèles 2 et 2'.

Dans le fixateur externe montré à la figure 1, la partie 4 de la tige 2 et l'ouverture réalisée dans l'élément mobile 9 ont une section telle que ce dernier est immobile en rotation autour de l'axe 8 de la partie 4 de la tige 2. Cet élément 9 est disposé sur la partie 4 susdite de la tige 2 de manière mobile et peut être remplacé par un autre élément 9 mieux approprié à la trépanation à effectuer.

Dans la forme de réalisation du fixateur externe 1 illustrée à la figure 2, l'élément mobile 9 présente, sur sa face 15 tournée vers l'élément écarteur 5, des ergots 16 dont les pointes sont destinées à pénétrer soit dans l'os 7 (figure 1), soit dans la plaque 12 de protection de la muqueuse 10 (figure 1), pour immobiliser parfaitement l'élément 9 et de là le fixateur externe 1 par rapport audit os 7 ou à la plaque de protection 12.

Comme montré à la figure 3, l'élément écarteur 5 présente avantageusement, sur sa face 17 tournée vers l'élément mobile 9 et pour encore renforcer l'immobilisation du fixateur 1 par rapport à l'os 7, deux plaques fixes 18 se raccordant à l'élément écarteur 5 suivant deux lignes 19 sensiblement parallèles au plan passant par les axes des tiges 2 et 2' et s'écartant régulièrement l'une de l'autre par rapport à un plan de trace 20, qui est parallèle au plan passant par les axes des tiges susdites, ces plaques 18 prenant appui sur l'os 7 de part et d'autre de la face 6 de ce dernier avec laquelle coopère l'élément écarteur 5.

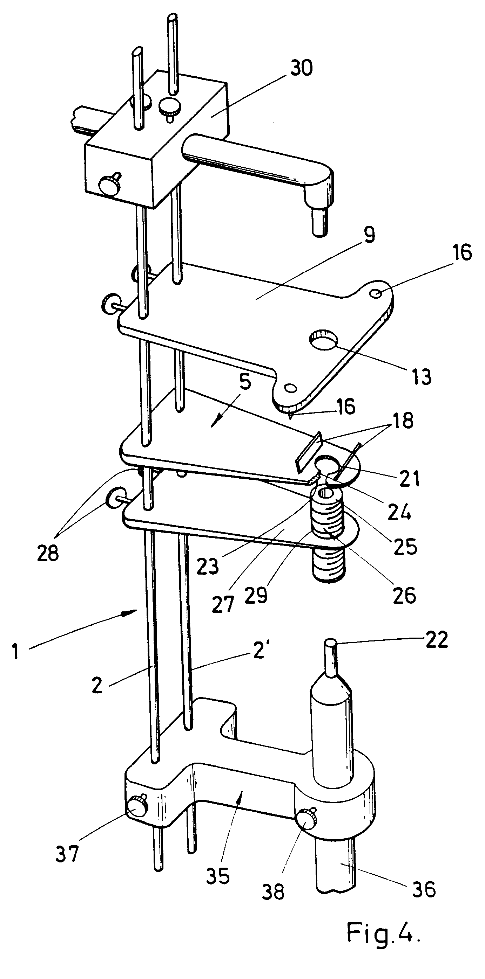

Toujours comme montré à la figure 3 ainsi qu'aux figures 2, 4 et 5, l'élément écarteur 5 présente, pour permettre la pose précise d'implants qui nécessite une trépanation de l'os 7 à partir des faces 6 et 11 de ce dernier, une ouverture 21 qui est coaxiale à l'ouverture 13 présentée par l'élément mobile 9, cette ouverture 21 permettant le passage d'un outil de trépanation, tel que fraise 22 (voir figure 4).

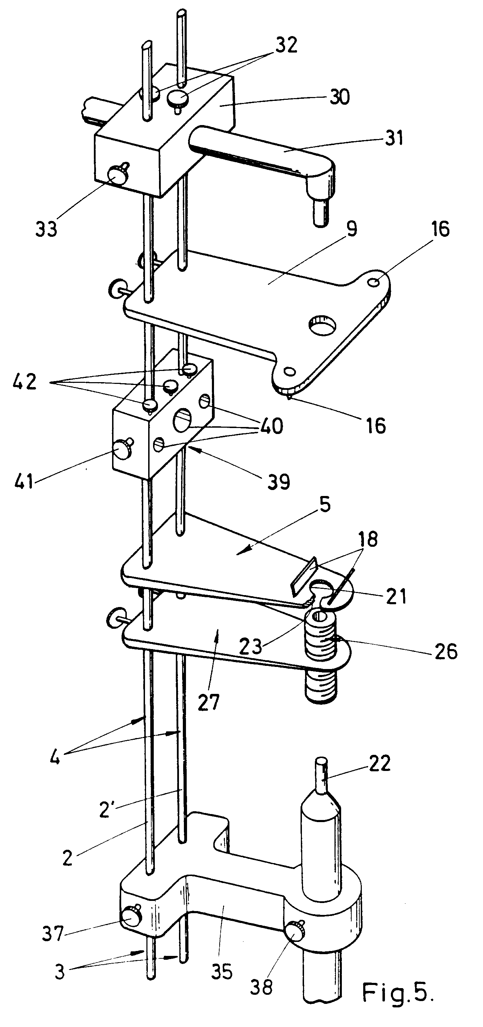

Pour assurer un guidage parfait de la fraise ou trépan 22 lorsque celui-ci est piloté manuellement, l'élément écarteur 5 présente, à partir de sa face 23 (figure 4), un évidement 24 coaxial à son ouverture et agencé pour recevoir, afin de l'immobiliser, l'extrémité 25 d'un guide 26, coaxial à ladite ouverture 21, à travers lequel passe la fraise 22. Ce guide 26 est avantageusement supporté, comme montré aux figures 4 et 5, par un organe mobile 27 qui coulisse librement sur la partie 4 des tiges 2 et 2', à l'opposé de l'élément mobile 9 par rapport à l'élément écarteur 5, des vis de pression 28 étant prévues pour immobiliser cet organe 27 par rapport aux tiges 2 et 2' lorsque l'extrémité 25 du guide 26 est logée dans l'évidement 23 prévu dans l'élément écarteur 5. Ce guide 26 est agencé sur l'organe mobile 27 pour que sa position soit réglable suivant une direction parallèle à l'axe des parties 4 des tiges 2 et 2' et est avantageusement constitué par un tube dans laquel la fraise ou le trépan 22 est introduit pour que son axe coïncide avec l'axe du tube, celui-ci étant fileté extérieurement et coopérant avec un trou taraudé 29 présenté par l'organe 27 et qui est coaxial aux ouvertures 13 et 21, le réglage précis de la position du guide 26 par rapport à l'organe 27 s'effectuant en faisant tourner le tube fileté qui le constitue autour de son axe, les filets du tube et du trou taraudé étant tels que le tube reste immobile en rotation autour de son axe tant qu'il n'est pas commandé positivement en rotation.

Pour que la fraise ou trépan 14 soit parfaitement guidé à travers l'ouverture 13 de l'élément mobile 9, le fixateur externe 1 comprend, comme montré aux figures 3 à 5, un support mobile 30 pour l'outil 14, porté par un contre-angle porte-fraise 31, qui est agencé pour coulisser librement le long des parties 4 des tiges 2 et 2', à l'opposé de l'élément écarteur 5 par rapport à l'élément mobile 9. Des vis de pression 32 et 33 sont prévues, d'une part, pour immobiliser le contre-angle porte-fraise 31 dans le logement 34 du support 30 pour que la fraise ou trépan 14 et l'ouverture 13 de l'élément mobile 9 soient coaxiales et, d'autre part, pour immobiliser en position choisie, quand on le désire, ledit support 30 par rapport aux tiges 2.

Quand on désire guider mécaniquement la fraise ou le trépan 22, on peut prévoir sur le fixateur 1, comme montré aux figures 4 et 5, un support mobile 35 pour une pièce à main portefraise 36 qui est agencé pour coulisser librement le long des tiges 2 et 2', à l'opposé de l'élément écarteur 5 par rapport à l'organe mobile 27. Des vis de pression 37 et 38 sont prévues pour immobiliser, quand on le désire et en position choisie, le support mobile 35 par rapport aux tiges 2 et 2' et pour immobiliser la pièce à main 36 pour que la fraise ou trépan 22 ait son axe qui coïncide avec l'axe de l'ouverture 21 pratiquée dans l'élément écarteur 5 et avec l'axe du guide 26.

Pour permettre une pose précise d'implants à verrouillage transversal ou de vis de fixation transversales du fixateur externe coopérant avec l'os, le fixateur externe 1 comprend avantageusement, comme montré à la figure 5, un support mobile 39 présentant des ouvertures 40, pour des fraises ou trépans non représentés, dont les axes sont parallèles et situés dans un plan perpendiculaire aux axes des tiges 2 et 2'. Les trépans guidés dans ces ouvertures 40 permettent de réaliser des trépanations qui, lorsqu'elles sont réunies entre elles, autorisent la pose d'éléments à ailettes immobilisant les implants. Le support 39, qui est agencé entre les éléments 5 et 9, porte des vis de pression 41 et 42 permettant, d'une part, d'immobiliser le support, en position choisie, par rapport aux tiges 2 et 2' et, d'autre part, d'immobiliser, quand on le désire, les trépans dans les ouvertures 40.

A part l'élément écarteur 5, qui est fixé aux tiges 2 et 2', tous les autres éléments 9, 27, 30, 35 et 39 sont avantageusement assemblés auxdites tiges 2 et 2' de manière amovible afin de pouvoir être utilisés ensemble ou séparément en fonction des trépanations à effectuer.

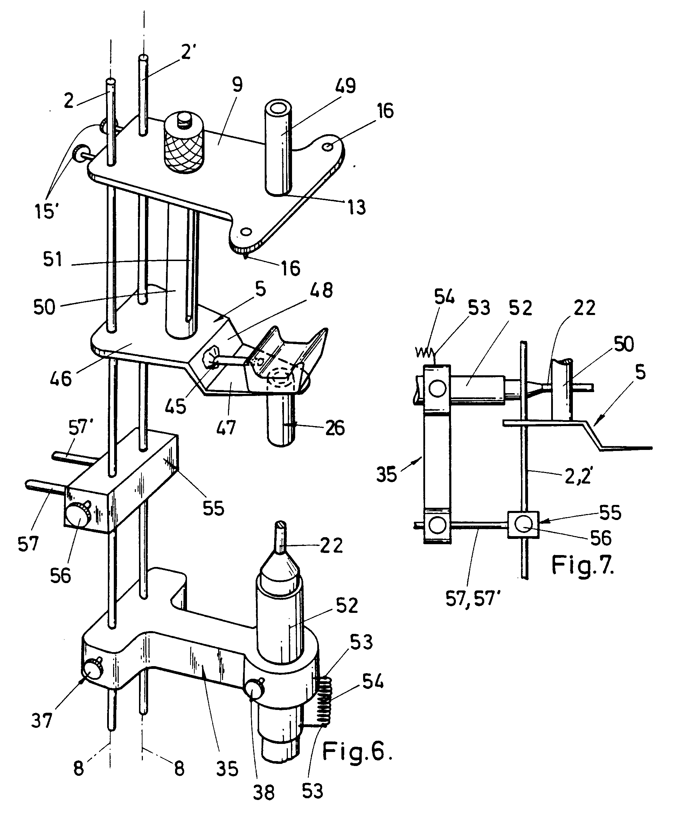

Le fixateur externe suivant l'invention et représenté aux figures 6 et 7 est plus particulièrement une variante du fixateur montré à la figure 5 et duquel l'organe mobile 27 a été supprimé, cet organe pouvant, dans certains cas, constituer une gêne pour l'écartement de lèvre, et dans lequel le support moibile 39 qui a été remplacé par un support mobile 55 destiné à recevoir le support 35 du trépan ou de la fraise 22, pour réaliser des trépanations suivant une direction perpendiculaire ou transversale au plan passant par les axes des tiges 2 et 2'.

Dans cette dernière forme de réalisation, l'élément écarteur 5, pour faciliter son introduction en regard de l'os 7, est coudé et est constitué de deux parties 46, 47, sensiblement planes et sensiblement perpendiculaires aux axes 8 des tiges 2 et 2', raccordées par un pan oblique 48, la partie 47, la plus éloignée des axes 8 et qui est destinée à être placée en regard de l'os, étant située à un niveau inférieur à la partie 46. Cette partie 47 est agencée pour supporter un moulage 44 de la partie de l'os, tel que le moulage réalisé à partir d'informations fournies par un scanner, sur laquelle l'écarteur doit prendre appui. Des moyens de fixation 45 de ce moulage, qui est amovible et différent pour chaque cas de trépanation sont agencés pour que ce moulage puisse épouser la partie d'os qu'il reproduit afin d'immobiliser totalement le fixateur externe par rapport à l'os lorsque ce dernier est pincé entre le moulage fixé à l'élément écarteur 5 et l'élément mobile 9 précité. De sorte que tout risque de déplacement intempestif du fixateur en cours de trépanation est écarté.

Pour guider d'une manière parfaite l'outil de trépanation, le guide 26, qui était, dans la forme de réalisation montrée à la figure 5, fixé à l'organe 27, est fileté et fixé directement sur l'élément écarteur 5 dans l'ouverture 21, qui est taraudée.

Pour guider parfaitement un outil, analogue à l'outil 31 susdit, l'ouverture 13 de l'élément mobile 9 est également avantageusement munie d'un guide tubulaire 49, coaxial à ladite ouverture 13, dont l'extrémité filetée est vissée dans cette ouverture 13 qui est taraudée.

Pour permettre une immobilisation totale du fixateur externe par rapport à l'os, celui-ci comprend une pièce de liaison 50 agencée entre l'élément écarteur 5 et l'élément mobile 9. Cette pièce est parallèle à l'axe 8 des tiges 2 et 2' et est agencée de manière à permettre, d'une part, le réglage de l'écartement entre l'élément écarteur 5 et l'élément mobile 9 et, d'autre part, l'immobilisation totale de ces deux éléments sur l'os.

Ladite pièce de liaison 50 présente une boutonnière 51 parallèle aux axes 8 précités et qui est destinée à livrer passage, par exemple, à l'outil de trépanation 22, comme expliqué ci-dessous.

Le support mobile 35 de ce fixateur externe supportant l'outil de trépanation 22 comprend un fourreau 52, agencé pour recevoir et immobiliser l'outil 22, coulissant librement dans ledit support mobile, des moyens 38 étant prévus sur ce dernier et agencés pour permettre l'immobilisation du fourreau 52 par rapport au support mobile 35 lors des manipulations et du transport du fixateur.

Le support mobile 35 et le fourreau 52 présentent avantageusement chacun une patte d'attache 53, pour une extrémité d'un ressort interchangeable 54 dont l'axe est sensiblement parallèle à l'axe du fourreau, ce ressort, choisi en fonction des caractéristiques de l'os faisant progresser automatiquement le fourreau dans le support 35 pour la trépanation.

Ce fixateur externe comprend aussi un support mobile 55 agencé pour coulisser librement le long des tiges 2 et 2' au voisinage de l'élément écarteur 5, à l'opposé de l'élément mobile 9. Des moyens 56 sont agencés pour immobiliser ce support 55 sur les tiges 2 et 2' en position choisie. Deux tiges 57 et 57', analogues aux tiges 2 et 2', sont fixées audit support 55 perpendiculairement à ces dernières. Sur ces tiges 57 et 57' peut coulisser et être immobilisé, comme montré à la figure 7, le support mobile 35 pour outil de trépanation 22, lorsque ce dernier est utilisé pour une trépanation à travers la boutonnière 51 précitée.

Il doit être entendu que l'invention n'est nullement limitée aux formes de réalisation décrites et que bien des modifications peuvent être apportées à ces dernières sans sortir du cadre du présent brevet.

## Revendications

1. Fixateur externe (1) pour chirurgie osseuse, en particulier chirurgie dento-maxilo-faciale, et notamment pour la réalisation de trépanations permettant la mise en place d'implants, comprenant au moins une tige (2) indéformable servant notamment de poignée au fixateur et présentant une partie (4) de section constante et d'axe rectiligne, un élément (5) écarteur, tel qu'écarteur de lèvre, destiné à prendre appui sur une première face (6) de l'os (7) à trépaner, qui est fixé à la tige à distance des extrémités de la partie susdite de cette dernière et sensiblement perpendiculairement à l'axe (8) de cette partie (4) de tige, au moins un élément mobile (9), associé à la partie de tige précitée de manière à pouvoir coulisser librement le long de cette dernière pour prendre appui soit sur la muqueuse (10) couvrant une seconde face (11) de l'os (7) opposée à la première avec laquelle coopère l'élément écarteur (5) ou sur une plaque de protection (12) de cette muqueuse, soit avec cette seconde face (11) de l'os (7), dans lequel est pratiquée une ouverture (13), d'axe sensiblement parallèle à l'axe (8) de la partie (4) de tige, destinée à livrer passage à un outil (14) de trépanation de l'os, tel qu'une fraise, et des moyens (15') prévus sur ledit élément pour l'immobiliser en position choisie par rapport à la partie (4) de tige précitée.

2. Fixateur externe suivant la revendication 1, caractérisé en ce que l'élément écarteur (5) fixé à la partie (4) de tige susdite présente une ouverture (21), d'axe sensiblement parallèle à l'axe (8) de ladite partie (4) de tige, destinée à livrer passage à un outil de trépanation (22), tel que fraise, les distances séparant l'axe de cette ouverture (21) et l'axe de l'ouverture (13) présentée par l'élément mobile (9) susdit de l'axe (8) de ladite partie de tige étant égales.

3. Fixateur externe suivant l'une ou l'autre des revendications 1 et 2, caractérisé en ce que l'élément écarteur (5) fixé à la tige comprend, disposés en saillie par rapport à sa face (17) tournée vers l'élément mobile (9) précité, des moyens fixes (18) profilés pour prendre appui sur l'os (7) de part et d'autre de la première face (6) précitée de ce dernier avec laquelle coopère ledit élément écarteur (5).

4. Fixateur externe suivant l'une ou l'autre des revendications 1 et 2, caractérisé en ce que l'élément écarteur (5) susdit est agencé pour supporter un moulage (44) de la partie de l'os, tel que moulage réalisé à partir d'informations fournies par un scanner, sur laquelle l'écarteur doit prendre appui et comprend des moyens de fixation (45) de ce moulage, qui est amovible et différent pour chaque cas de trépanation, qui sont agencés pour que ce moulage puisse épouser la partie d'os qu'il reproduit afin d'immobiliser totalement le fixateur externe par rapport à l'os lorsque ce dernier est pincé entre le moulage fixé à l'élément écarteur (5) et l'élément mobile (9) précité.

5. Fixateur externe suivant l'une ou l'autre des revendications 3 et 4, caractérisé en ce que l'élément écarteur (5) susdit présente, à partir de sa face (24) opposée à celle sur laquelle sont disposés les moyens fixes (18) profilés précités ou le moulage (44), un évidement (24) coaxial à l'ouverture (21) pratiquée dans ledit élément et agencé pour recevoir, pour l'immobiliser, l'extrémité (25) d'un guide (26), coaxial à ladite ouverture (21), pour un outil de trépanation.

6. Fixateur externe suivant la revendication 4, caractérisé en ce que l'évidement (24) coaxial susdit est taraudé de manière à pouvoir y visser, pour l'immobiliser sur l'élément écarteur (5), l'extrémité (25) du guide (26) précité.

7. Fixateur externe suivant l'une quelconque des revendications 1 à 6, caractérisé en ce que l'élément écarteur (5) est coudé et est constitué de deux parties (46, 47), sensiblement planes et sensiblement perpendiculaires à l'axe (8) de la partie (4) de tige susdite, raccordées par un pan oblique (48), la partie (47), la plus éloignée de l'axe (8) et qui est destinée à être placée en regard de l'os, étant située à un niveau inférieur à l'autre partie (46).

8. Fixateur externe suivant l'une quelconque des revendications 1 à 7, caractérisé en ce que l'élément mobile (9) susdit présente, sur sa face (15) tournée vers l'élément écarteur (5) précité, des moyens, tels qu'au moins un ergot (16), agencés pour pénétrer soit dans l'os (7), soit dans la plaque (12) susdite protégeant la muqueuse (10) afin d'immobiliser ledit élément mobile (9) par rapport à cet os (7) ou plaque (12).

9. Fixateur externe suivant l'une quelconque des revendications 1 à 7, caractérisé en ce que l'ouverture (13) présentée par l'élément mobile (9) est taraudée de manière à pouvoir y fixer l'extrémité filetée d'un guide tubulaire (49) coaxial à ladite ouverture (13).

10. Fixateur externe suivant l'une quelconque des revendications 1 à 9, caractérisé en ce qu'il comprend une pièce de liaison (50) entre l'élément écarteur (5) et l'élément mobile (9), cette pièce étant parallèle à l'axe (8) de la partie (4) de la tige et agencée de manière à permettre, d'une part, le réglage de l'écartement entre l'élément écarteur (5) et l'élément mobile (9) et, d'autre part, l'immobilisation totale de ces éléments sur l'os (7).

11. Fixateur externe suivant la revendication 10, caractérisé en ce que la pièce de liaison (50) susdite présente une boutonnière (51) parallèle à l'axe (8) précité et destinée à livrer passage à un outil de trépanation.

12. Fixateur externe suivant l'une quelconque des revendications 1 à 11, caractérisé en ce qu'il comprend un support mobile (30) pour un outil de trépanation (14), tel que contre-angle porte-fraise (31), agencé pour coulisser librement le long de la partie (4) de tige (2) précitée à l'opposé de l'élément écarteur (5) par rapport à l'élément mobile (9) susdit, des moyens (33) agencés pour immobiliser le support mobile (9) par rapport à la tige (2) en position choisie, des moyens (32) pour emprisonner le contre-angle porte-fraise ou trépan et des moyens pour l'immobiliser par rapport audit support mobile pour que la fraise et l'ouverture (13) réalisée dans l'élément mobile (9) soient coaxiales.

13. Fixateur externe suivant l'une quelconque des revendications 5 à 12, caractérisé en ce qu'il comprend un organe mobile (27) destiné à supporter le guide (26) précité pour l'outil de trépanation afin que ce guide et l'ouverture (13) pratiquée dans l'élément écarteur (5) soient coaxiaux, cet organe coulissant librement sur la partie précitée (4) de tige à l'opposé de l'élément mobile (9) par rapport à l'élément écarteur (5) et présentant des moyens (28) agencés pour l'immobiliser sur la partie (4) de tige en position choisie.

14. Fixateur externe suivant la revendication 13, caractérisé en ce que le guide (26) susdit est agencé sur l'organe mobile (27) de manière à pouvoir être déplacé suivant une direction parallèle à l'axe de la partie (4) de tige (2) précitée, des moyens étant prévus pour l'immobiliser par rapport audit organe (27) en position choisie.

15. Fixateur externe suivant la revendication 14, caractérisé en ce que le guide (26) est constitué par un tube dans lequel est introduit l'outil de trépanation (22) pour que l'axe de ce dernier coïncide avec l'axe du tube, ce tube étant fileté extérieurement et coopérant avec un trou taraudé (29) présenté par l'organe mobile (27), ce trou taraudé étant coaxial, d'une part, aux ouvertures (21, 13) présentées par l'élément écarteur (5) et par l'élément mobile (9) précités, et, d'autre part, à l'outil de trépanation immobilisé dans le support mobile susdit.

16. Fixateur externe suivant l'une quelconque des revendications 7 à 9, caractérisé en ce qu'il comprend un support mobile (35) pour outil de trépanation (22), tel que pièce à main portefraise, agencé pour coulisser librement le long de la partie (4) de tige (2) précitée, à l'opposé de l'élément écarteur (5), par rapport à l'organe mobile (27) susdit, des moyens (37) agencés pour immobiliser ce support, en position choisie, par rapport à la partie de tige précitée et des moyens (38) agencés pour immobiliser l'outil de trépanation (22) pour que son axe coïncide avec l'axe de l'ouverture (21) pratiquée dans l'élément écarteur (5).

17. Fixateur externe suivant la revendication 16, caractérisé en ce que le support mobile (35) pour outil de trépanation (22) comprend un fourreau (52), agencé pour recevoir et immobiliser l'outil (22), coulissant librement dans ledit support mobile, des moyens (38) étant prévus sur ce dernier et agencés pour permettre l'immobilisation du fourreau (52) par rapport au support mobile (35).

18. Fixateur externe suivant la revendication 17, caractérisé en ce que le support mobile (35) et le fourreau (52) présentent chacun une patte d'attache (53) pour une extrémité d'un ressort interchangeable (54) dont l'axe est sensiblement parallèle à l'axe du fourreau.

19. Fixateur externe suivant l'une quelconque des revendications 16 à 18, caractérisé en ce qu'il comprend un support mobile (55) agencé pour coulisser librement le long de la partie (4) de tige (2) précitée au voisinage de l'élément écarteur (5), à l'opposé de l'élément mobile (9), des moyens (56) agencés pour immobiliser ce support (55) sur la partie (4) de tige (2) en position choisie et au moins une tige (57) fixée audit support (55) perpendiculairement à la partie (4) de tige (2), sur laquelle peut coulisser et être immobilisé le support mobile (35) pour outil de trépanation (22).

20. Fixateur externe suivant l'une quelconque des revendications 1 à 19, caractérisé en ce qu'il comprend un support mobile (39) pour au moins un outil de trépanation, tel que fraise, agencé pour coulisser librement le long de la partie (4) de tige (2) précitée dans sa section comprise entre l'élément écarteur (5) et l'élément mobile (9) précités, des moyens (41) agencés pour immobiliser ce support, en position choisie, par rapport à la partie de tige et des moyens (42) pour immobiliser l'outil de trépanation dans le support pour que son axe soit transversal à l'axe de l'ouverture pratiquée dans l'élément écarteur.

21. Fixateur externe suivant la revendication 20, caractérisé en ce que la partie (4) de tige (2) précitée et les ouvertures réalisées dans l'élément mobile (9), l'organe mobile (27) et les quatre supports (30, 35, 39 et 55) pour leur permettre de coulisser librement sur cette partie (4) de tige (2), ont une section telle que lesdits éléments (9) et organe (27) mobiles ainsi que les quatre supports sont immobiles en rotation autour de l'axe (8) de la partie (4) de tige (2) susdite.

22. Fixateur externe suivant la revendication 20, caractérisé en ce qu'il comprend deux tiges parallèles (2, 2') sur lesquelles peuvent coulisser librement les éléments (9) et organe (27) mobiles précités ainsi que les quatre supports (30, 35, 39 et 55) susdits.

23. Fixateur externe suivant l'une ou l'autre des revendications 21 et 22, caractérisé en ce que les éléments (9) et organe (27) mobiles et les quatre supports (30, 35, 39 et 55) précités sont disposés sur la tige (2) ou les tiges (2, 2') de manière amovible.

## Claims

1. External fixator (1) for bone surgery, in particular for dentomaxillofacial surgery and particularly for carrying out trepanations that allow for installing implants, comprising at least one dimensionally stable rod (2) that serves, in particular, as a grip to the fixator and that displays one portion (4) of constant section and rectilinear axis, one spreading element (5) such as a lip spreader destined to rest on a first facies (6) of the bone (7) to be trepaned, which is fitted to the rod at a distance from the ends of the said portion of the latter, and substantially perpendicular to the axis (8) of this portion (4) of the rod, at least one mobile element (9) joined to the said rod portion in such a manner as to be able to slide freely along the latter until it comes to rest either on the mucous membrane (10) covering a second facies (11) of the bone (7) opposite to the first facies with which co-operates the spreading element (5) or on a plate (12) protecting this mucous membrane, or with this second facies (11) of the bone (7), in which is made an opening (13) whose axis is substantially parallel to the axis (8) of the rod portion (4), destined to leave the way open to a bone trepanning tool (14) such as a drill, and means (15') provided at the said element for the purpose of fixing it in the position choses in relation to the said portion (4) of the rod.

2. External fixator according to claim 1, characterized in that the spreading element (5) fitted to the said rod portion (4) displays an opening (21) whose axis is substantially parallel to the axis (8) of the said rod portion (4) and which is destined to leave the way open to a trepanning tool (22) such as a drill, with the distances separating the axis of this opening from the axis of the opening (13) displayed by the said mobile element (9) of the said rod portion being equal.

3. External fixator according to either one of the claims 1 and 2, characterized in that the spreading element (5) fitted to the rod comprises, in a projecting arrangement in relation to its face (17) turned towards the said mobile element (9), fixed means (18) profiled so as to rest on the bone (7) at both sides of the said facies (6) of the latter with which co-operates the said spreading element (5).

4. External fixator according to either one of the claims 1 and 2, characterized in that the said spreading element (5) is designed so as to sustain a cast (44) of the bone portion such as a cast made in accordance with data provided by a scanner, and on which the spreader will have to rest, and that it comprises means for securing (45) this cas which is interchangeable and different for each case of trepanation, which means are designed so that this cast can fit exactly the bone portion it is reproducing in order to totally fix in position the external fixator in relation to the bone when the latter is gripped between the cast fitted to the spreading element (5) and the said mobile element (9).

5. External fixator according to either one of the claims 3 and 4, characterized in that the said spreading element (5) displays at its face (24) opposite to the face on which are arranged the said profiled fixed means (18) or the cast (44), a hollow (24) that is coaxial to the opening (21) made in the said element and designed to take, for the purpose of fixing it in position, the end (25) of a guide (26) coaxial to the said opening (21), for a trepanning tool.

6. External fixator according to claim 4, characterized in that the said coaxial hollow (24) is tapped so as to be able to screw and thus fix in position on the spreading element (5), the end of the said guide (26).

7. External, fixator according to either one of the claims 1 to 6, characterized in that the spreading element (5) is bent and consists of two parts (46, 47) that are substantially plane and substantially perpendicular to the axis (8) of the said rod portion (4) and connected through an oblique section (48), with the part (47) farthest removed from the axis (8) and destined to be installed opposite the bone located at a level lower as compared to the other part (46).

8. External fixator according to either one of the claims 1 to 7, characterized in that the said mobile element (9) displays at its face (15) turned towards the said spreading element (5), means such as at least one peg (16) destined to penetrate either into the bone (7) or into the said plate (12) that protects the mucous membrane (10) for the purpose of fixing in position the said mobile element (9) in relation to this bone (7) or plate (12).

9. External fixator according to either one of the claims 1 to 7, characterized in that the opening (13) displayed by the mobile element (9) is tapped in such a manner as to allow for fitting therein the threaded end of a tubular guide (49) coaxial to the said opening.

10. External fixator according to either one of the claims 1 to 9, characterized in that it comprises a joining part (50) between the spreading element (5) and the mobile element (9), this part being parallel to the axis (8) of the portion (4) of the rod and designed in such a manner as to allow, on one hand, the adjustment of the clearance between the spreading element (5) and the mobile element (9) and, on the other hand, the total fixing in position of these elements on the bone (7).

11. External fixator according to claim 10, characterized in that the said joining part (50) displays a slot (51) parallel to the said axis (8) and destined to leave the way open to a trepanning tool.

12. External fixator according to either one of the claims 1 to 11, characterized in that it comprises a mobile holder (30) for a trepanning tool (14), such as a counterangle drill holder (31), designed to slide freely along the portion (4) of the said rod (2) opposite the spreading element (5), in relation to the said mobile element (9), also means (33) designed to fix in position the mobile element (9) in relation to the rod (2), in the position chosen, and means (32) for the purpose of confining the counterangle drill holder or trepan and means to fix it in position in relation to the said mobile holder so that the drill and the opening (13) made in the mobile element (9) are coaxial.

13. External fixator according to either one of the claims 5 to 12, characterized in that it comprises a mobile component (27) destined to hold the said guide (26) for the trepanning tool so that this guide and the opening (13) made in the spreading element (5) are coaxial, with this component sliding freely on the said portion (4) of the rod opposite to the mobile element (9) in relation to the spreading element and displaying means (28) designed to fix it in the position chosen on the portion (4) of the rod.

14. External fixator according to claim 13, characterized in that the said guide (26) is arranged on the mobile component (27) in such a manner as to allow for being displaced in a direction parallel to the axis of the portion (4) of the said rod (2), with means being provided for the purpose of fixing it in the chosen position in relation to the said component (27).

15. External fixator according to claim 14, characterized in that the guide (26) consists of a tube into which is inserted the trepanning tool (22) so that the axis of the latter coincides with the axis of the tube, with this tube being threaded on the outside and co-operating with a tapped hole (29) displayed by the mobile component (27), with this tapped hole being coaxial, on one hand, to the openings (21, 13) displayed by the spreading element (5) and by the said mobile element (9) and, on the other hand, to the trepanning tool fixed in position in the said mobile holder.

16. External fixator according to either one of the claims 7 to 9, characterized in that it comprises a mobile holder (35) for a trepanning tool (22) such as a handheld drill holder designed to slide freely along the said portion (4) of the rod (2), opposite the spreading element (5), in relation to the said mobile component (27), and means (37) designed to fix in the chosen position this holder, in relation to the said rod portion, and means (38) designed to fix in position the trepanning tool (22) so that its axis coincides with the axis of the opening (21) made in the spreading element (5).

17. External fixator according to claim 16, characterized in that the mobile holder (35) for the trepanning tool (22) comprises a sleeve (52) designed to take and fix in position the tool (22) that slides freely in the said mobile holder and means (38) provided on the latter and designed to allow for fixing in position the sleeve (52) in relation to the mobile holder (35).

18. External fixator according to claim 17, characterized in that the mobile holder (35) and the sleeve (52) each display a securing flange (53) for one end of an interchangeable spring (54) whose axis is substantially parallel to the axis of the sleeve.

19. External fixator according to either one of the claims 16 to 18, characterized in that it comprises a mobile holder (55) designed to slide freely along the said portion (4) of the rod (2), in the neighbourhood of the spreading element (5), opposite to the mobile element (9), and means (56) designed to fix in the chosen position this holder (55) on the portion (4) of the rod (2), and at least one rod (57) fitted to the said holder (55) in perpendicular to the portion (4) of the rod (2) on which can slide freely and become fixed in position the mobile holder (35) for the trepanning tool (22).

20. External fixator according to either one of the claims 1 to 19, characterized in that it comprises one mobile holder (39) for at least one trepanning tool such as a drill, designed to slide freely along the said portion (4) of the rod (2) in its section comprised between the spreading element (5) and the said mobile element (9), and means (41) designed to fix this holder in the chosen position in relation to the rod portion, and means (42) for fixing in position the trepanning tool in the holder, so that its axis is transversal to the axis of the opening made in the spreading element.

21. External fixator according to claim 20, characterized in that the said portion (4) of the rod (2) and the openings made in the mobile element (9), the mobile component (27) and the four holders (30, 35, 39 and 55) display, in order to enable them to slide freely on this portion (4) of the rod (2), a section such that the said mobile elements (9) and component (27) as well as the four holders are fixed in position, in rotation, around the axis (8) of the said portion (4) of the rod (2).

22. External fixator according to claim 20, characterized in that it comprises two parallel rods (2, 2') on which can slide freely the said mobile elements (9) and component (27) as well as the said four holders (30, 35, 39 and 55).

23. External fixator according to either one of the claims 21 and 22, characterized in that the mobile elements (9) and component (27) as well as the said four holders (30, 35, 39 and 55) are interchangeably arranged on the rod (2) or the rods (2, 2').

## Patentansprüche

1. Äußerliche Fixiervorrichtung (1) für die Knochenchirurgie, insbesondere für die Zahn-, Kiefer- und Gesichtschirurgie und hier speziell für die Durchführung Von Trepanationen, mit denen Implantate eingesetzt werden können, bestehend aus mindestens einem unverformbaren Schaft (2), der insbesondere als Griff der Fixiervorrichtung dient und einen Teil (4) mit konstantem Querschnitt und geradliniger Achse aufweist, einem Spreizelement (5), zum Beispiel einem Lippenspreizer, das sich auf eine erste Fläche (6) des zu trepanierenden Knochens (7) abstützen kann und das in einem Abstand von den Enden des genannten Teils des Schafts und im wesentlichen senkrecht zur Achse (8) dieses Teils (4) des Schafts am Schaft befestigt ist, mindestens einem beweglichen Element (9), das so mit dem vorgenannten Schaftteil verbunden ist, daß es frei entlang dieses Teils gleiten kann, so daß es sich entweder auf der Schleimhaut (10), die eine zweite, der ersten Fläche, mit der das Spreizelement (5) zusammenwirkt, gegenüberliegenden Fläche (11) des Knochens (7) bedeckt, oder auf einer Schutzplatte (12) für diese Schleimhaut abstützt oder aber auf dieser zweiten Fläche (11) des Knochens (7), in die eine Öffnung (13) eingearbeitet ist, deren Achse im wesentlichen parallel zur Achse (8) des Schaftteils (4) verläuft und den Durchgang für ein Werkzeug (14), zum Beispiel einen Fräser, zur Trepanation des Knochens freigibt, und auf dem genannten Element vorgesehenen Mitteln (15') zur Immobilisierung in einer gewünschten Lage relativ zum vorgenannten Schaftteil (4).

2. Äußerliche Fixiervorrichtung gemäß Anspruch 1,
**dadurch gekennzeichnet,**
daß das am Schaftteil (4) befestigte Spreizelement (5) eine Öffnung (21) besitzt, deren Achse im wesentlichen parallel zur Achse (8) des Schaftteils (4) verläuft und den Durchgang für ein Trepanationswerkzeug (22), zum Beispiel einen Fräser, freigibt, wobei die Abstände von der Achse dieser Öffnung (21) und von der Achse der Öffnung (13) im beweglichen Element (9) zur Achse (8) des genannten Schaftteils gleich sind.

3. Äußerliche Fixiervorrichtung gemäß Anspruch 1 oder 2,
**dadurch gekennzeichnet,**
daß das am Schaft befestigte Spreizelement (5) feste, profilierte Mittel (18) besitzt, die aus seiner dem beweglichen Element (9) zugewandten Fläche (17) herausragen und sich zu beiden Seiten der ersten Fläche (6) des Knochens, mit der das Spreizelement (5) zusammenwirkt, am Knochen (7) abstützen.

4. Äußerliche Fixiervorrichtung gemäß Anspruch 1 oder 2,
**dadurch gekennzeichnet,**
daß das Spreizelement (5) so angeordnet ist, das es eine Abformung (44), zum Beispiel eine Abformung, die aufgrund der von einem Scanner gelieferten Informationen erstellt wurde, desjenigen Knochenteils trägt, auf den sich das Spreizelement abstützen soll, und Befestigungsmittel (45) für diese Abformung beinhaltet, die abnehmbar und je nach durchzuführender Trepanation unterschiedlich ist, wobei diese Befestigungsmittel So angeordnet sind, daß diese Abformung sich dem Teil des Knochens, dessen Form sie hat, anschmiegen kann, um die äußerliche Fixiervorrichtung in Bezug auf den Knochen völlig zu immobilisieren, wenn dieser zwischen der am Spreizelement (5) befestigten Abformung und dem beweglichen Element (9) eingeklemmt ist.

5. Äußerliche Fixiervorrichtung gemäß Anspruch 3 oder 4,
**dadurch gekennzeichnet,**
daß das Spreizelement (5), ausgehend von seiner Fläche (24), die der Fläche, auf der sich die festen, profilierten Mittel (18) oder die Abformung (44) befinden, gegenüberliegt, eine Aussparung (24) besitzt, die koaxial zu der in das genannte Element eingearbeitete Öffnung (21) ist und so angeordnet ist, daß sie das Ende (25) einer zu der Öffnung (21) koaxialen Führung (26) für ein Trepanationswerkzeug aufnehmen kann, um dieses Ende zu immobilisieren.

6. Äußerliche Fixiervorrichtung gemäß Anspruch 4,
**dadurch gekennzeichnet,**
daß die koaxiale Aussparung (24) mit einem Innengewinde versehen ist, so daß dort das Ende (25) der Führung (26) eingeschraubt werden kann, so daß es auf dem Spreizelement (5) immobilisiert wird.

7. Äußerliche Fixiervorrichtung gemäß einem der Ansprüche 1 bis 6,
**dadurch gekennzeichnet,**
daß das Spreizelement (5) abgewinkelt ist und aus zwei Teilen (46, 47) besteht, die im wesentlichen eben sind und im wesentlichen auf der Achse (8) des Schaftteils (4) senkrecht stehen und durch eine schräge Fläche (48) miteinander verbunden sind, wobei sich der von der Achse (8) weiter entfernte Teil (47), der dem Knochen zugewandt plaziert werden soll, auf niedrigerer Höhe befindet als der andere Teil (46).

8. Äußerliche Fixiervorrichtung gemäß einem der Ansprüche 1 bis 7,
**dadurch gekennzeichnet,**
daß das bewegliche Element (9) auf seiner Fläche (15), die dem Spreizelement (5) zugewandt ist, Mittel, zum Beispiel mindestens einen Haken (16) besitzt, die so angeordnet sind, daß sie entweder in den Knochen (7) oder in die die Schleimhaut (10) schützende Platte (12) eindringen, um das bewegliche Element (9) in Bezug auf diesen Knochen (7) oder dieser Platte (12) zu immobilisieren.

9. Äußerliche Fixiervorrichtung gemäß einem der Ansprüche 1 bis 7,
**dadurch gekennzeichnet,**
daß die Öffnung (13) in dem beweglichen Element (9) mit einem Innengewinde versehen ist, so daß dort das gewindete Ende einer röhrenförmigen, zur Öffnung (13) koaxialen Führung (49) eingeschraubt werden kann.

10. Äußerliche Fixiervorrichtung gemäß einem der Ansprüche 1 bis 9,
**dadurch gekennzeichnet,**
daß sie ein Verbindungsstück (50) zwischen dem Spreizelement (5) und dem beweglichen Element (9) beinhaltet, wobei dieses Stück parallel zur Achse (8) des Schaftteils (4) verläuft und so angeordnet ist, daß es einerseits die Einstellung des Abstands zwischen dem Spreizelement (5) und dem beweglichen Element (9) und andererseits die vollständige Immobilisierung dieser Elemente auf dem Knochen (7) ermöglicht.

11. Äußerliche Fixiervorrichtung gemäß Anspruch 10,
**dadurch gekennzeichnet,**
daß das Verbindungsstück (50) eine zur Achse (8) parallel verlaufende längliche Aussparung (51) besitzt, die dazu dient, den Durchgang für ein Trepanationswerkzeug freizugeben.

12. Äußerliche Fixiervorrichtung gemäß einem der Ansprüche 1 bis 11,
**dadurch gekennzeichnet,**
daß sie eine bewegliche Auflage (30) für ein Trepanationswerkzeug (14), zum Beispiel einen Fräserträgerwinkel (31), beinhaltet, die so angeordnet ist, daß sie frei entlang des Teils (4) des Schafts (2) gleiten kann, der auf der dem beweglichen Element (9) gegenüberliegenden Seite des Spreizelements (5) liegt, Mittel (33), die so angeordnet sind, daß sie die bewegliche Auflage (30) relativ zum Schaft (2) in einer gewünschten Position immobilisieren, Mittel (32) zum Festhalten des Fräser- oder Bohrerhalterwinkels und der Immobilisierungsmittel relativ zur genannten beweglichen Auflage, damit der Fräser und die Öffnung (13) im beweglichen Element (9) koaxial sind.

13. Äußerliche Fixiervorrichtung gemäß einem der Ansprüche 5 bis 12,
**dadurch gekennzeichnet,**
daß sie ein bewegliches Teil (27) beinhaltet, das dazu dient, die Führung (26) für das Trepanationswerkzeug zu halten, damit diese Führung und die Öffnung (21) im Spreizelement (5) koaxial sind, wobei dieses Teil frei entlang des Schaftteils (4) gleiten kann, der auf der dem Spreizelement (5) gegenüberliegenden Seite des beweglichen Elements (9) liegt, und Mittel (28) besitzt, die so angeordnet sind, daß sie das Teil (27) auf dem Schaftteil (4) in einer gewünschten Position immobilisieren.

14. Äußerliche Fixiervorrichtung gemäß Anspruch 13,
**dadurch gekennzeichnet,**
daß die Führung (26) so auf dem beweglichen Teil angeordnet ist, daß sie in einer Richtung parallel zur Achse des Teils (4) des Schafts (2) verschoben werden kann, wobei Mittel vorgesehen sind, um die Führung in einer gewünschten Position in Bezug auf den beweglichen Teil (27) zu immobilisieren.

15. Äußerliche Fixiervorrichtung gemäß Anspruch 14,
**dadurch gekennzeichnet,**
daß die Führung (26) aus einer Röhre besteht, in die das Trepanationswerkzeug (22) so eingeführt wird, daß seine Achse mit der Achse der Röhre zusammenfällt, wobei diese Röhre ein Außengewinde hat, das mit einem im beweglichen Teil (27) vorhandenen Gewindeloch (29) zusammenwirkt, wobei dieses Loch einerseits zu den Öffnungen (21, 13) im Spreizelement (5) bzw. im beweglichen Element (9) und andererseits zu dem in der genannten beweglichen Auflage immobilisierten Trepanationswerkzeug koaxial ist.

16. Äußerliche Fixiervorrichtung gemäß einem der Ansprüche 7 bis 9,
**dadurch gekennzeichnet,**
daß sie eine bewegliche Auflage (35) für das Trepanationswerkzeug (22) beinhaltet, zum Beispiel ein Fräserträger-Handstück, die so angeordnet ist, daß sie frei entlang des Teils (4) des Schafts (2) gleiten kann, der auf der dem Spreizelement (5) gegenüberliegenden Seite des beweglichen Teils (27) liegt, Mittel (37), die so angeordnet sind, daß sie diese Auflage in Bezug auf den genannten Schaftteil in einer gewünschten Position immobilisieren, und Mittel (38) die so angeordnet sind, daß sie das Trepanationswerkzeug (22) so immobilisieren, daß seine Achse mit der Achse der in das Spreizelement (5) eingearbeiteten Öffnung (21) zusammenfällt.

17. Äußerliche Fixiervorrichtung gemäß Anspruch 16,
**dadurch gekennzeichnet,**
daß die bewegliche Auflage (35) für das Trepanationswerkzeug (22) eine Hülse (52) beinhaltet, die so angeordnet ist, daß sie das im der genannten beweglichen Auflage frei gleitende Trepanationswerkzeug (22) aufnimmt und immobilisiert, wobei an dieser Auflage Mittel (38) vorgesehen und so angeordnet sind, daß die Hülse (52) in Bezug auf die bewegliche Auflage (35) immobilisiert werden kann.

18. Äußerliche Fixiervorrichtung gemäß Anspruch 17,
**dadurch gekennzeichnet,**
daß die bewegliche Auflage (35) und die Hülse (52) Jeweils einen Befestigungshaken (53) für die Enden einer auswechselbaren Feder (54) aufweisen, deren Achse sich im wesentlichen parallel zur der Achse der Hülse erstreckt.

19. Äußerliche Fixiervorrichtung gemäß einem der Ansprüche 16 bis 18,
**dadurch gekennzeichnet,**
daß sie eine bewegliche Auflage (55) beinhaltet, die so angeordnet ist, daß sie in der Nähe des Spreizelements (5) und auf dessen dem beweglichen Element (9) gegenüberliegenden Seite frei entlang des Teils (4) des Schafts (2) gleitet, Mittel (56), die so angeordnet sind, daß sie diese Auflage in einer gewünschten Position auf dem Teil (4) des Schafts (2) immobilisieren, und mindestens einem senkrecht zum Teil (4) des Schafts (2) an der genannten Auflage (55) befestigten Schaft (57), auf dem die bewegliche Auflage (55) für das Trepanationswerkzeug (22) gleiten und immobilisiert werden kann.

20. Äußerliche Fixiervorrichtung gemäß einem der Ansprüche 1 bis 18,
**dadurch gekennzeichnet,**
daß sie eine bewegliche Auflage (39) für mindestens ein Trepanationswerkzeug, zum Beispiel einen Fräser, beinhaltet, die so angeordnet ist, daß sie in dem Abschnitt zwischen dem Spreizelement (5) und dem beweglichen Element (9) frei entlang des Teils (4) des Schafts (2) gleitet, Mittel (41), die so angeordnet sind, daß sie diese Auflage in einer gewünschten Position auf dem Schaftteil immobilisieren, und Mittel (42), um das Trepanationswerkzeug so in der Auflage Zu immobilisieren, daß seine Achse quer zur Achse der Öffnung im Spreizelement verläuft.

21. Äußerliche Fixiervorrichtung gemäß Anspruch 20,
**dadurch gekennzeichnet,**
daß der Teil (4) des Schafts (2) und die Öffnungen, die in das bewegliche Element (9), den beweglichen Teil (27) und die vier Auflagen (30, 35, 39 und 55) eingearbeitet sind, damit diese frei auf dem Teil (4) des Schafts (2) gleiten können, einen solchen Querschnitt haben, daß sich das bewegliche Element (9), der bewegliche Teil (27) sowie die vier Auflagen nicht um die Achse (8) des Teils (4) des Schafts (2) drehen können.

22. Äußerliche Fixiervorrichtung gemäß Anspruch 20,
**dadurch gekennzeichnet,**
daß sie zwei parallele Schäfte (2, 2') beinhaltet, auf denen das bewegliche Element (9), der bewegliche Teil (27) und die vier Auflagen (30, 35, 39 und 55) frei gleiten können.

23. Äußerliche Fixiervorrichtung gemäß einem der Ansprüche 21 und 22,
**dadurch gekennzeichnet,**
daß das bewegliche Element (9), der bewegliche Teil (27) und die vier Auflagen (30, 35, 39 und 55) abnehmbar an dem Schaft (2) oder den Schäften (2, 2') angebracht sind.
